Europäisches Patentamt

(19) European Patent Office

Office européen des brevets

(11) Veröffentlichungsnummer: **0 041 974**
**B1**

(12) # EUROPÄISCHE PATENTSCHRIFT

(45) Veröffentlichungstag der Patentschrift:
31.10.84

(51) Int. Cl.³: **A 61 B 17/18**

(21) Anmeldenummer: **80902399.7**

(22) Anmeldetag: **12.12.80**

(86) Internationale Anmeldenummer:
**PCT/AT 80/00035**

(87) Internationale Veröffentlichungsnummer:
**WO 81/01647 (25.06.81 Gazette 81/15)**

(54) **INSTRUMENTARIUM ZUR REPOSITION UND FIXATION VON PER- UND SUBTROCHANTEREN FRAKTUREN SOWIE EINEN TEIL DIESES INSTRUMENTARIUMS BILDENDES EINSATZSTÜCK.**

(30) Priorität: **14.12.79 AT 7896/79**

(43) Veröffentlichungstag der Anmeldung:
**23.12.81 Patentblatt 81/51**

(45) Bekanntmachung des Hinweises auf die Patenterteilung:
**31.10.84 Patentblatt 84/44**

(84) Benannte Vertragsstaaten:
**CH DE FR GB LI NL SE**

(56) Entgegenhaltungen:
**CH - A - 458 615**
**DE - A - 2 115 289**
**US - A - 4 169 470**

(73) Patentinhaber: **Ender, Hans Georg, Dr.,**
**Ferstelgasse 6/20, A-1090 Wien (AT)**

(72) Erfinder: **Ender, Hans Georg, Dr., Ferstelgasse 6/20,**
**A-1090 Wien (AT)**

(74) Vertreter: **Brauneiss, Leo et al, Patentanwälte Dipl.-Ing.**
**Peter Boeckmann, Dipl.-Ing. Leo Brauneiss**
**Strohgasse 10, A-1030 Wien (AT)**

## Beschreibung

Die Erfindung betrifft ein Instrumentarium zur Reposition und Fixation von per- und subtrochanteren Frakturen, mit wenigstens einem zumindest in seinem proximalen Endbereich gekrümmten Knochennagel aus elastischem Material, der über ein proximal des Kniegelenkbereiches im Knochen angeordnetes Einschlagloch in den Markraum des Knochens einführbar ist und infolge seiner Elastizität mit dem Krümmungsscheitel an der dem Einschlagloch gegenüberliegenden Wand des Markraumes unter Spannung anliegt, und der an seinem distalen Ende mit einem Kupplungsteil versehen ist, der eine allseits drehfeste Verbindung mit einem Einschlaggerät ermöglicht. Ein derartiges Instrumentarium ist aus der US-A-4 169 470 bekannt. Ferner betrifft die Erfindung ein Einsatzstück, welches einen Teil dieses Instrumentariums bildet.

Es ist aus oben genanntem Dokument bereits bekannt, per- und subtrochantere Frakturen dadurch zu reponieren und zu fixieren, daß der Markraum des Knochens durch ein Einschlagloch eröffnet wird und in dieses Einschlagloch zumindest ein Knochennagel, zweckmäßig mehrere Knochennägel, eingeführt werden, die aus einem elastischen Material bestehen und wenigstens in ihrem proximalen Bereich gekrümmt sind. Werden diese Knochennägel in den Markraum eingeführt, so liegen sie infolge ihrer Elastizität mit dem Krümmungsscheitel an der dem Einschlagloch gegenüberliegenden Wand des Markraumes unter Spannung an, wobei dann, wenn die proximale Spitze jedes Nagels in den Bereich der Bruchstelle kommt, der Nagel über diese Bruchstelle in den Gelenkskopf des Knochens eintritt und die Bruchstelle fixiert. Durch Drehen der einzelnen Nägel können die Knochenteile reponiert werden, so daß sie an der Bruchstelle ihre richtige Lage relativ zueinander einnehmen. Hierzu ist das distale Ende jedes Nagels mit einem Kupplungsteil versehen, der eine allseits drehfeste Verbindung mit einem Einschlaggerät ermöglicht. Es ist bereits bekannt, diesen Kupplungsteil als plättchenförmige Abflachung auszubilden, es sind jedoch auch andere Ausbildungen des Kupplungsteiles möglich. Befinden sich die bekannten Nägel in der richtigen Lage im Markraum, so ragen die Nagelenden aus dem Einschlagloch heraus und der Kupplungsteil liegt an der Außenseite des Knochens distal des Einschlagloches unter Spannung an, wodurch nicht nur insbesondere bei älteren Personen mit porotischen Knochen die Gefahr besteht, daß der Knochen an der Anliegestelle einbricht, sondern auch die für über dem Einschlagloch laufenden Sehnen und Muskel durch die herausragenden distalen Nagelenden irritiert werden.

Das Einschlagloch wird üblicherweise so hergestellt, daß zunächst der Knochen punktförmig eröffnet und anschließend daß so gebildete kleine Loch durch einen gebogenen, drei- oder vierkantigen Pfriem aufgerieben, durch einen Meißel vergrößert oder mit einem Bohrer aufgebohrt wird. In allen diesen Fällen kann es zu einem Absplittern des Knochens und dadurch zu einer unerwünschten Vergrößerung des Einschlagloches kommen. Aber auch beim Einschlagen der Nägel kommt es zeitweise durch die tangentiale Scherbeanspruchung an der proximalen Kortikalis zum Absplittern eines Kortikaliskeiles, was zu einer unerwünschten Vergrößerung des Einschlagloches führt, wobei der Rand des Einschlagloches auch an der Vorderseite einbrechen kann. In beiden Fällen folgt daraus ein unkontrollierbares Abstehen der Nagelenden und es kann , wenn sich der Bruch in den Knochen hinein fortsetzt, sogar ein durch den Operateur hervorgerufener Drehbruch des Oberschenkels entstehen.

Es kann aber auch vorkommen, daß die Knochennägel zu tief in das Einschlagloch eingeschlagen werden, so daß der Kupplungsteil nicht mehr an der Knochenaußenseite anliegt, oder daß der an der Knochenaußenseite anliegende Kupplungsteil durch Ausbrechen eines Teiles des Lochrandes sich gegen das Knocheninnere verschiebt.

Gelangt der Kupplungsteil in das Innere des Markraumes und verschwindet somit im Knochen, so tritt auf jeden Fall der Nachteil auf, daß die erforderliche Spannung der Nägel nachläßt und daher der angestrebte Effekt nicht mehr gewährleistet ist. Befindet sich der Nagel zur Gänze im Markraum, so kann es weiters vorkommen, daß er sich an den im Markraum befindlichen Spongiosabälgchen verfängt und dadurch daran gehindert wird, nach distal zu rutschen. Wird in diesem Fall der Knochen belastet, so daß die Knochenteile an der Bruchstelle hierdurch einander genähert werden, so kann es sein, daß die Nagelspitze den Gelenkskopf des Knochens perforiert und in die Hüftpfanne eindringt. Werden die zur Gänze im Markraum angeordneten Nägel nicht von den Spongiosabälgchen zurückgehalten, so rutschen die Nägel nach distal und können dann nur äußerst schwer wieder entfernt werden. Jedenfalls ist es zum Entfernen solcher Nägel notwendig, das Einschlagloch derart zu vergrößern, daß das sich im Markraum befindliche distale Ende erfaßt werden kann.

Die vorliegende Erfindung hat sich zur Aufgabe gestellt, diese Nachteile zu vermeiden. Die Erfindung geht hierbei aus von einem Instrumentarium der eingangs beschriebenen Art und besteht im wesentlichen darin, daß ein in das Einschlagloch einsetzbares Einsatzstück vorgesehen ist, das einen Führungskanal für den Knochennagel bzw. die Knochennägel aufweist und dessen Kupplungsteil zumindest teilweise aufnimmt, und das mit einer seine Verschiebung im Einschlagloch verhindernden Fixiereinrichtung versehen ist. Dieses Einsatzstück, welches in das Einschlagloch eingesetzt und in diesem durch die Fixiereinrichtung festgelegt ist, verhindert zunächst beim Einschlagen der Nägel, daß das Einschlagloch aussplittert und dadurch in nachteiliger Weise vergrößert wird, es wird vielmehr die Größe der Öffnung, durch welche die Knochennägel in den Markraum eingeschlagen werden, genau durch den Querschnitt des Führungskanales bestimmt. Außerdem ermöglicht es die Verwendung des Einsatzstückes, die Nägel so tief in den Knochen einzuschlagen, daß ihr distales Ende vom Einsatzstück aufgenommen wird und daher nicht mehr herausragt, so daß eine Irritation der Muskel und Sehnen verhindert wird, jedoch auch ein Eindringen der mit dem Kupplungsteil versehenen Enden der Nägel in den Markraum vermieden wird. Die von den Kupplungsteilen der unter Spannung stehenden Nägel ausgeübten Kräfte werden vielmehr vom Einsatzstück aufgenommen und von diesem gleichmäßig entlang des ganzen Umfanges des Einschlagloches auf den Knochen übertragen, so daß auch die Gefahr eines Einbrechens des Knochens im Bereich des Einschlagloches selbst bei älteren Menschen mit porotischen Knochen nicht mehr gegeben ist. Schließlich werden beim Einschlagen die Nägel an der Einschlagstelle durch den Führungskanal des Einsatzstückes geführt, so daß auch bei diesem Einschlagen nicht die Gefahr einer Beschädigung des Knochens durch die tangentiale Scherbeanspruchung an der proximalen Kortikalis gegeben ist. Da die mit den Kupplungsteilen versehenen distalen Enden der Nägel im Einsatzstück angeordnet und daher leicht erreichbar sind, können bei dem erfindungsgemäßen Instrumentarium die Nägel auch leicht wieder aus dem Markraum entfernt werden.

Wie bereits erwähnt, muß das Einsatzstück mit einer Fixiereinrichtung versehen sein, die seine Verschiebung also ein Herausfallen des Einsatzstückes aus dem Einschlagloch oder ein zu weites Eindringen in den Markraum verhindert. Gemäß einer bevorzugten Ausführungsform der Erfindung besteht daher das Einsatzstück aus einem rohrförmigen oder rinnenförmigen Abschnitt, der einen nach außen abstehenden Flansch aufweist, der an der Knochenaußenseite anliegt. Dieser Flansch wirkt als Anschlag und verhindert beim Anliegen an der Knochenaußenseite eine weitere Verschiebung des Einsatzstückes in Richtung zum Markraum. Ist der Abschnitt rohrförmig ausgebildet, so umschließt er die distalen Enden der Knochennägel zur Gänze. Da jedoch diese Enden durch die Spannung der Knochennägel ohnedies nur im distalen Bereich des Abschnittes anliegen, genügt es auch, diesen Abschnitt rinnenförmig, also nach proximal offen, auszubilden, ohne daß hierbei der angestrebte Effekt wesentlich beeinträchtigt wird.

Vorzugsweise schließt die Achse des rohrförmigen oder rinnenförmigen Abschnittes mit der Flanschebene einen spitzen Winkel ein, so daß bei entsprechender Anordnung des Einsatzstückes im Einschlagloch der vom rohrförmigen oder rinnenförmigen Abschnitt begrenzte Führungskanal eine solche Lage aufweist, daß die eingeschlagenen Nägel ihre richtige Stellung im Markraum einnehmen. Damit die richtige Stellung des Einsatzstückes sichergestellt ist, ist es auch erforderlich, sowohl eine Verdrehung als auch ein Herausrutschen dieses Einsatzstückes vor allem auch beim Einschlagen der Nägel zu verhindern. Zweckmäßig weist daher das Einsatzstück mindestens einen an der Wand des Markraumes anliegenden Ansatz auf, der sowohl ein unerwünschtes Herausfallen des Einsatzstückes aus dem Einschlagloch als auch durch Reibungsschluß eine unerwünschte Verdrehung des Einsatzstückes verhindert. Diese Ansätze können beispielsweise von federnden Zungen gebildet sein, die beim Einsetzen des Einsatzstückes in das Einschlagloch nachgeben, sobald jedoch das Einsatzstück sich in seiner richtigen Lage befindet, ausfedern und den Rand des Einschlagloches hintergreifen. Zweckmäßig sind diese Zungen von aus dem rohrförmigen oder rinnenförmigen Abschnitt herausgebogenen Teilen gebildet. Die Zungen können dann über den ganzen Umfang verteilt sein und sind leicht herstellbar, wobei hierfür beispielsweise nur Ausstanzungen im rohrförmigen oder rinnenförmigen Abschnitt vorgenommen werden müssen. Es kann aber auch der den Führungskanal bildende Teil des Einsatzstückes nach Art eines bekannten Spreizdübels ausgebildet sein, so daß sich dieser Teil bei entsprechender Betätigung im Schlagloch verspreizt und daher das Einsatzstück weder verdreht noch entfernt werden kann.

Schließlich ist es im einfachsten Fall möglich, im Flansch zumindest eine Öffnung zum Hindurchführen einer im Knochen verankerbaren Schraube od. dgl. vorzusehen, durch welche gleichfalls eine Sicherung gegen unerwünschtes Verdrehen und unerwünschtes Entfernen des Einsatzstückes gegeben ist.

Um ein unerwünschtes Austreten der distalen Nagelenden aus dem Einsatzstück mit Sicherheit zu verhindern, kann gemäß einem weiteren Merkmal der Erfindung der Führungskanal des Einsatzstückes durch einen Verschluß verschließbar sein. Dieser Verschluß kann beispielsweise aus einem Deckel bestehen, der vorzugsweise mit einem in den Führungskanal hineinragenden Kragen versehen ist. Um eine sichere Verbindung dieses Deckels mit dem Einsatzstück zu gewährleisten, kann der Deckel mit dem Einsatzstück über einen Bajonettverschluß verbindbar sein oder mit einem Gewinde versehen sein, das mit einem im Einsatzstück angeordneten Gewinde zusammenwirkt. Es ist auch möglich, den Kragen konisch auszubilden und mit Preßsitz im Führungskanal zu befestigen.

Weiters ist es möglich, den Führungskanal des Einsatzstückes dadurch zu verschließen, daß dieser Führungskanal nach dem Einsetzen der Nägel zumindest teilweise mit einem erhärtendem Material, vorzugsweise mit aushärtbarem Kunststoffmaterial, ausgießbar ist. Dadurch werden die distalen Nagelenden in ihrer Lage so fixiert, daß sie keinerlei Verschiebungen mehr vornehmen können. Um eine sichere Verankerung dieses Materiales nach dem Erhärten im Führungskanal zu gewährleisten, ist dieser Erfindungsgemäß in dem auszugießenden Teil mit Erhebungen, wie Rillen od. dgl. versehen.

In der Zeichnung ist die Erfindung an Hand von Ausführungsbeispielen schematisch veranschaulicht.

Fig. 1 zeigt ein erfindungsgemäßes Instrumentarium, das in einem eine Fraktur aufweisenden Knochen angeordnet ist.

Fig. 2 stellt das Einsatzstück eines erfindungsgemäßen Instrumentariums in Vorderansicht dar, und

Fig. 3 zeigt einen Schnitt nach der Linie III-III der Fig. 2.Die

Fig. 4 bis 7 zeigen abgewandelte Ausführungsformen des Einsatzstückes in einem Schnitt entsprechend dem in Fig. 3 dargestellten Schnitt.

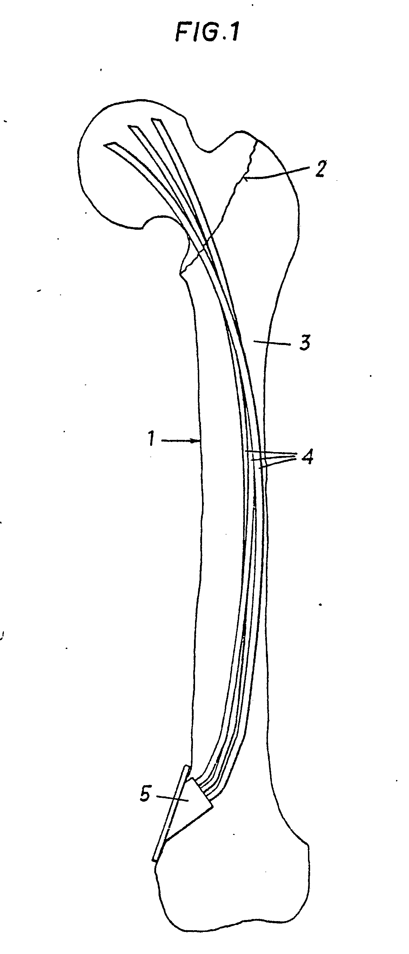

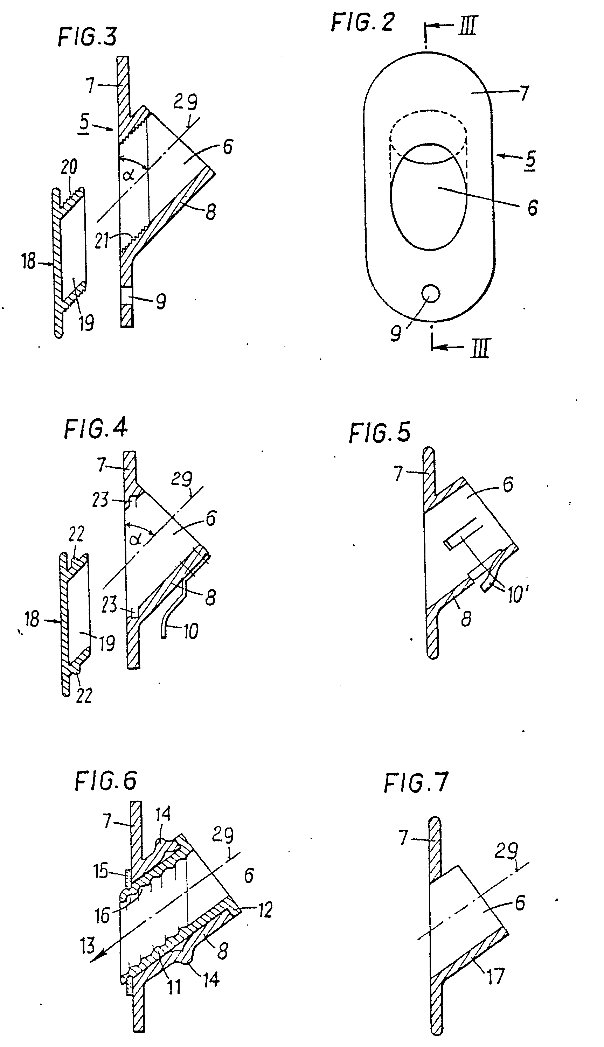

In Fig. 1 ist ein Knochen 1 gezeigt, der mit einer Fraktur 2 versehen ist. Zur Reposition und Fixation der Fraktur 2 sind im Markraum 3 des Knochens 1 drei aus elastischem Material bestehende, im proximalen Endbereich gekrümmte Knochennägel 4 eingesetzt. Hierfür wird zunächst der Markraum im Gelenkbereich des Knochens punktförmig eröffnet und anschließend mit einem Fräser oder Bohrer ein Einschlagloch gefräst oder gebohrt, wobei die Lochachse so gewählt wird, daß das Einschlagen der Nägel 4 in der notwendigen Richtung erfolgen kann. Anschließend wird ein Einsatzstück 5 in das Einschlagloch eingesetzt, worauf die Knochennägel 4 eingeschlagen und verdreht werden, um die Bruchstelle zu reponieren. Hierzu sind die Knochennägel an ihrem distalen Ende mit einem nicht dargestellten Kupplungsteil versehen, der eine allseits drehsichere Verbindung mit einem Einschlaggerät ermöglicht. Zweckmäßig ist dieser Kupplungsteil von einer plättchenförmigen Abflachung des distalen Nagelendes gebildet und mit einem Schlitz versehen, über welchen das Entfernen der eingesetzten Nägel möglich ist. Das Einsatzstück 5 weist einen Führungskanal 6 (siehe Fig. 2 bis 7) auf, der so angeordnet ist, daß die Nägel beim Einschlagen in der gewünschten Weise in den Markraum 3 einlaufen. Außerdem ist das Einsatzstück 5 mit einem Flansch 7 versehen, der an der Knochenaußenseite anliegt und ein zu weites Eindringen des Einsatzstückes 5 in den Markraum verhindert. Die Nägel 4 werden so weit eingeschlagen, daß ihr distales, mit dem Kupplungsteil versehenes Ende vom Führungskanal aufgenommen wird, also nicht nach außen vorsteht und die darüberliegenden Sehnen und Muskel irritiert, aber auch nicht in den Markraum 3 hineingleitet.

Bei den Ausführungsformen nach den Fig. 3 bis 6 ist der Führungskanal 6 von einem mit dem Flansch 7 verbundenen Rohrstück 8 und bei der Ausführungsform nach Fig. 7 ist der Führungskanal von einem rinnenförmigen Abschnitt 17 gebildet, der nach proximal offen ist. Die Achse 29 dieses Rohrstückes 8 bzw. des rinnenförmigen Abschnittes 17 schließt mit der Ebene des Flansches 7 einen spitzen Winkel $\alpha$ ein, so daß der Führungskanal 6 bei richtig in das Einschlagloch eingesetztem Einsatzstück 5 die gewünschte Lage zum Einführen der Knochennägel 4 einnimmt. Damit diese Lage beibehalten wird, ist es zweckmäßig, das Einsatzstück 5 sowohl gegen Verdrehung als auch gegen unerwünschtes Herausgleiten aus dem Einschlagloch zu fixieren. Bei der Ausführungsform des Einsatzstückes nach den Fig. 2 und 3 ist hierbei in dem Flansch 7 eine Öffnung 9 vorgesehen, durch die eine Schraube hindurchgeführt werden kann, die im Knochen eingeschraubt wird. Es können auch mehrere Öffnungen mit mehreren Schrauben vorgesehen sein. Bei der Ausführungsform nach Fig. 4 weist das Einsatzstück einen bei eingesetztem Einsatzstück an der Innenwand des Markraumes anliegenden Ansatz auf, der von einer federnden Zunge 10 gebildet ist. Diese federnde Zunge 10 ist mit ihrem einen Ende an der Außenseite des Rohrstückes 8 befestigt und an ihrem anderen Ende so ausgebildet, daß sie beim Einschieben des Einsatzstückes in das Einschlagloch sich an der Außenwand des Rohrabschnittes 8 anlegt, dann jedoch zurückfedert und etwa die in Fig. 4 gezeigte Lage einnimmt, in der sie den Rand des Einschlagloches hintergreift und dadurch eine Fixierung des Einsatzstückes 5 bewirkt.

Bei der Ausführungsform nach Fig. 5 sind mehrere federnde Zungen 10' vorgesehen, die von ausgestanzten Herausbiegungen des Rohrstückes 8 gebildet sind. Dadurch lassen sich die federnden Zungen 10' sehr rationell herstellen.

Bei der Ausführungsform nach Fig. 6 ist im Innern des Rohrabschnittes 8 eine Hülse 11 angeordnet, die mit einem Ansatz 12 versehen ist. Der Rohrabschnitt 8 und zweckmäßig auch der Flansch 7 sind bei dieser Ausführungsform aus nachgiebigem Material, beispielsweise aus Kunststoff, hergestellt. Nach dem Einsetzen des Einsatzstückes 5 im Einschlagloch wird die Hülse 11 in Richtung des Pfeiles 13 angezogen, wodurch sich der Rohrabschnitt 8 bei 14 aufweitet und das Einschlagloch an seiner Innenseite hintergreift, so daß dadurch das Einsatzstück fixiert wird. Die Lage der Hülse 11 kann beispielsweise durch einen Sprengring od. dgl. 15 festgelegt werden.

Um ein Austreten der distalen Nagelenden aus dem Einsatzstück 5 mit Sicherheit zu verhindern, ist es zweckmäßig, dieses Einsatzstück zu verschließen. Dies kann beispielsweise dadurch geschehen, daß in das Einsatzstück ein erhärtendes Material, beispielsweise ein aushärtbares Kunststoffmaterial, eingegossen wird. Dieses Material hält nach seiner Erhärtung die distalen Nagelenden unverrückbar fest. Um eine gute Bindung dieses Materiales nach seiner Erhärtung mit dem Führungskanal 6 zu gewährleisten, ist dieser Führungskanal zumindest teilweise mit Erhebungen, vorzugsweise Rillen 16, versehen, wie dies in Fig. 6 bei der Hülse angedeutet ist.

Es kann aber auch der Führungskanal 6 einfach durch einen Deckel 18 verschlossen werden, wie dies in den Fig. 3 und 4 angedeutet ist.

Bei der Ausführungsform nach Fig. 3 ist der Deckel 18 mit einem Kragen 19 versehen, der ein Außengewinde 20 aufweist, das mit einem Innengewinde 21 im Führungskanal zusammenwirkt. Bei der Ausführungsform nach Fig. 4 weist der Kragen 19 abstehende Zapfen 22 auf, die in bajonettverschlußartige Ausnehmung 23 im Flansch 7 einsetzbar sind. Es kann aber auch der Kragen 19 lediglich konisch ausgebildet sein und mit Preßsitz in den Führungskanal 6 eingesetzt sein. In allen Fällen wird eine sichere Verankerung des Deckels 18 im Einsatzstück 5 gewährleistet.

Bei allen Ausführungsformen kann das Einsatzstück aus rostfreiem Stahl oder aus Kunststoff oder aus einer Kombination dieser Werkstoffe bestehen.

## Patentansprüche

1. Instrumentarium zur Reposition und Fixation von per- und substrochanteren Frakturen, mit wenigstens einem zumindest in seinem proximalen Endbereich gekrümmten Knochennagel (4) aus elastischem Material, der über ein proximal des Kniegelenkbereiches im Knochen (1) angeordnetes Einschlagloch in den Markraum (3) des Knochens (1) einführbar ist und infolge seiner Elastizität mit dem Krümmungsscheitel an der dem Einschlagloch gegenüberliegenden Wand des Markraumes (3) unter Spannung anliegt, und der an seinem distalen Ende mit einem Kupplungsteil versehen ist, der eine allseits drehfeste Verbindung mit einem Einschlaggerät ermöglicht, dadurch gekennzeichnet, daß ein in das Einschlagloch einsetzbares Einsatzstück (5) vorgesehen ist, das einen Führungskanal (6) für den Knochennagel (4) bzw. die Knochennägel aufweist, dessen Seitenwand den vom Kupplungsteil des im Markraum (3) des Knochens (1) unter Spannung liegenden Nagels (4) auf den Knochen (1) ausgeübten Druck aufnimmt und über eine größere Fläche verteilt auf den Knochen (1) überträgt, und das mit einer seiner Verschiebung im Einschlagloch verhindernden Fixiereinrichtung (7, 9, 10, 14) versehen ist.

2. Instrumentarium nach Anspruch 1, dadurch gekennzeichnet, daß das Einsatzstück (5) aus einem rohrförmigen oder rinnenförmigen Abschnitt (8, 17) besteht, der einen nach außen abstehenden Flansch (7) aufweist, der an der Knochenaußenseite anliegt.

3. Instrumentarium nach Anspruch 1 und 2, dadurch gekennzeichnet, daß die Achse (29) des rohrförmigen oder rinnenförmigen Abschnittes (8, 17) mit der Flanschebene einen spitzen Winkel (α) einschließt.

4. Instrumentarium nach Anspruch 1, dadurch gekennzeichnet, daß das Einsatzstück (5) mindestens einen an der Wand des Markraumes anliegenden Ansatz (10, 10') aufweist.

5. Instrumentarium nach Anspruch 4, dadurch gekennzeichnet, daß der Ansatz von mindestens einer federnden Zunge (10, 10') gebildet ist (Fig. 4, 5).

6. Instrumentarium nach Anspruch 5, dadurch gekennzeichnet, daß die Zungen (10') von aus dem rohrförmigen oder rinnenförmigen Abschnitt (8, 17) herausgebogenen Teilen gebildet sind.

7. Instrumentarium nach Anspruch 1, dadurch gekennzeichnet, daß der den Führungskanal (6) bildende Teil des Einsatzstückes (5) nach Art eines Spreizdübels ausgebildet ist (Fig. 6).

8. Instrumentarium nach Anspruch 2, dadurch gekennzeichnet, daß im Flansch (7) zumindest eine Öffnung (9) zum Hindurchführen einer im Knochen verankerbaren Schraube vorgesehen ist (Fig. 2, 3).

9. Instrumentarium nach Anspruch 1, dadurch gekennzeichnet, daß der Führungskanal (6) des Einsatzstückes (5) durch einen Verschluß (18) verschließbar ist.

10. Instrumentarium nach Anspruch 9, dadurch gekennzeichnet, daß der Verschluß aus einem Deckel (18) besteht, der vorzugsweise mit einem in den Führungskanal (6) hineinragenden Kragen (19) versehen ist.

11. Instrumentarium nach Anspruch 10, dadurch gekennzeichnet, daß der Deckel (18) mit dem Einsatzstück (5) über einen Bajonettverschluß (22, 23) verbindbar ist.

12. Instrumentarium nach Anspruch 10, dadurch gekennzeichnet, daß der Deckel (18) mit einem Gewinde (20) versehen ist, das mit einem im Einsatzstück (5) angeordneten Gewinde (21) zusammenwirkt.

13. Instrumentarium nach Anspruch 10, dadurch gekennzeichnet, daß der Kragen (19) des Deckels (18) konisch ausgebildet ist und mit Preßsitz im Führungskanal (6) befestigt ist.

14. Instrumentarium nach Anspruch 9, dadurch gekennzeichnet, daß der Führungskanal (6) nach dem Einsetzen der Nägel (4) zumindest teilweise mit einem erhärtenden Material vorzugsweise mit aushärtbarem Kunststoffmaterial ausgießbar ist.

15. Instrumentarium nach Anspruch 14, dadurch gekennzeichnet, daß der Führungskanal (6) in dem auszugießenden Teil mit Erhebungen vorzugsweise Rillen (16), versehen ist.

16. Einsatzstück zur Verwendung in einem Instrumentarium nach Anspruch 1, dadurch gekennzeichnet, daß es einen Führungskanal (6) für den Knochennagel bzw. die Knochennägel (4) aufweist, dessen Seitenwandung den vom Kupplungsteil des im Markraum (3) des Knochens (1) unter Spannung liegenden Nagels (4) auf den Knochen ausgeübten Druck aufnimmt und über eine größere Fläche verteilt auf den Knochen (1) überträgt, und daß es mit einer seine Verschiebung im Einschlagloch verhindernden Fixiereinrichtung (7, 9, 10, 14) versehen ist.

17. Einsatzstück nach Anspruch 16, dadurch gekennzeichnet, daß es einen rohrförmigen oder rinnenförmigen Abschnitt (8, 17) und einen von diesem nach außen abstehenden Flansch (7) aufweist, welcher Flansch an der Knochenaußenseite anliegt.

18. Einsatzstück nach Anspruch 17, dadurch gekennzeichnet, daß die Achse (29) des rohrförmigen oder rinnenförmigen Abschnittes (8, 17) mit der Flanschebene einen spitzen Winkel ($\alpha$) einschließt.

19. Einsatzstück nach Anspruch 16, dadurch gekennzeichnet, daß es mindestens einen an der Wand des Markraumes anliegenden Ansatz (10, 10') aufweist.

20. Einsatzstück nach Anspruch 19, dadurch gekennzeichnet, daß der Ansatz von mindestens einer federnden Zunge (10, 10') gebildet ist (Fig. 4, 5).

21. Einsatzstück nach Anspruch 20, dadurch gekennzeichnet, daß die Zungen (10') von aus dem rohrförmigen oder rinnenförmigen Abschnitt (8, 17) herausgebogenen Teilen gebildet sind.

22. Einsatzstück nach Anspruch 16, dadurch gekennzeichnet, daß der den Führungskanal (6) bildende Teil nach Art eines Spreizdübels ausgebildet ist (Fig. 6).

23. Einsatzstück nach Anspruch 17, dadurch gekennzeichnet, daß im Flansch (7) mindestens eine Öffnung (9) zum Hindurchführen einer im Knochen verankerbaren Schraube vorgesehen ist (Fig. 2, 3).

24. Einsatzstück nach Anspruch 16, dadurch gekennzeichnet, daß sein Führungskanal (6) durch einen Verschluß (18) verschließbar ist.

25. Einsatzstück nach Anspruch 24, dadurch gekennzeichnet, daß der Verschluß aus einem Deckel (18) besteht, der vorzugsweise mit einem in den Führungskanal (6) hineinragenden Kragen (19) versehen ist.

26. Einsatzstück nach Anspruch 25, dadurch gekennzeichnet, daß der Deckel (18) über einen Bajonettverschluß (22, 23) mit dem Einsatzstück (5) verbindbar ist.

27. Einsatzstück nach Anspruch 25, dadurch gekennzeichnet, daß der Deckel (18) mit einem Gewinde (20) versehen ist, das mit einem im Einsatzstück (5) angeordneten Gewinde (21) zusammenwirkt.

28. Einsatzstück nach Anspruch 25, dadurch gekennzeichnet, daß der Kragen (19) des Deckels (18) konisch ausgebildet ist und mit Preßsitz im Führungskanal (6) befestigt ist.

29. Einsatzstück nach Anspruch 24, dadurch gekennzeichnet, daß der Führungskanal (6) nach dem Einsetzen der Nägel (4) zumindest teilweise mit einem erhärtenden Material, vorzugsweise mit aushärtbarem Kunststoffmaterial, ausgießbar ist.

30. Einsatzstück nach Anspruch 29, dadurch gekennzeichnet, daß der Führungskanal (6) in dem auszugießenden Teil mit Erhebungen vorzugsweise Rillen (16), versehen ist.

## Claims

1. Instrumentarium for repositioning and fixing pertrochanterous fractures, comprising at least one arcuate bone nail (4) of elastic material, said nail being arcuate at least at its proximal end portion and being insertable into the medullary canal (3) of this bone (1) via an impact hole arranged in the bone proximally of the knee joint and is, in view of its elasticity, contacting under tension with the apex of the arcuate portion the wall opposing the impact hole, of the medullary canal (3) and being provided at its distal end with a coupling portion allowing in all directions a positive connection for rotation with an impact tool, characterized that an insert member (5) is provided and member having a guiding channel (6) for the bone nail (4) or bone nails, respectively, the side wall of said guiding channel taking over the pressure exerted on the bone (1) by the coupling portion of the nail (4) lying in the medullary canal (3) of the bone (1) under tension, and transferring this pressure distributed over a larger area onto the bone (1), said insert member being provided with a fixing means (7, 9, 10, 14) preventing the insert member from becoming shifted within the impact hole.

2. Instrumentarium as claimed in claim 1, characterized in that the insert member (5) consists of a tubular or channel-shaped section (8, 17) having an outwardly protruding flange (7) contacting the outer surface of the bone.

3. Instrumentarium as claimed in claim 1 and 2, characterized in that the axis (29) of the tubular or channel-shaped section (8, 17) includes an acute angle ($\alpha$) with the plane of the flange.

4. Instrumentarium as claimed in claim 1, characterized in that the insert member (5) has at least one projection (10, 10') contacting the wall of the medullary canal.

5. Instrumentarium as claimed in claim 4, characterized in that the projection is formed by at least one resilient tongue (10, 10') (fig. 4, 5).

6. Instrumentarium as claimed in claim 5, characterized in that the tongues (10') are formed by parts bent out of the tubular or channel-shaped section (8, 17).

7. Instrumentarium as claimed in claim 1, characterized in that the part of the insert member (5), which forms the guiding channel (6), is designed in the manner of a straddling dowel (fig. 6).

8. Instrumentarium as claimed in claim 2, characterized in that at least one opening (9) is provided within the flange (7) for passing therethrough a screw adapted to be anchored within the bone (fig. 2, 3).

9. Instrumentarium as claimed in claim 1, characterized in that the guiding channel (6) of the insert member (5) can be closed by a closure means (18).

10. Instrumentarium as claimed in claim 9, characterized in that the closure means consists of a lid (18), preferably being provided with a coller (19) extending into the guiding channel (6).

11. Instrumentarium as claimed in claim 10, characterized in that the lid (18) can be connected with the insert member (5) by means of a bayonet lock (22, 23).

12. Instrumentarium as claimed in claim 10, characterized in that the lid (18) is provided with a thread (20) co-operating with a thread (21) provided in the insert member (5).

13. Instrumentarium as claimed in claim 10, characterized in that the collar (19) of the lid (18) is conically shaped and fixed within the guiding channel (6) by force-fitting.

14. Instrumentarium as claimed in claim 9, characterized in that the guiding channel (6) can, after inserting the nails (4), at least partially be filled by casting with a hardening material, preferably a setting synthetic plastics material.

15. Instrumentarium as claimed in claim 14, characterized in that the guiding channel (6) is provided with ele vations, preferably grooves (16), within the portion to be filled by casting.

16. Insert member for use in an instrumentarium according to claim 1, characterized in that it has a guiding channel (6) for the bone nail or bone nails (4), respectively, the side wall of said guiding channel taking over the pressure exerted on the bone by the coupling portion of the nail (4) lying in the medullary canal (3) of the bone (1) under tension, and transferring this pressure distributed over a larger area onto the bone (1), and in that it is provided with a fixing means (7, 9, 10, 14) preventing its shifting movement within the impact hole.

17. Insert member as claimed in claim 16, characterized in that it has a tubular or channel-shaped section (8, 17) having an outwardly protruding flange (7) contacting the outer surface of the bone.

18. Insert member as claimed in claim 17, characterized in that the axis (29) of the tubular or channel-shaped section (8, 17) includes an acute angle ($\alpha$) with the plane of the flange.

19. Insert member as claimed in claim 16, characterized in that it has at least one projection (10, 10') contacting the wall of the medullary canal.

20. Insert member as claimed in claim 19, characterized in that the projection is formed by at least one resilient tongue (10, 10') (fig. 4, 5).

21. Insert member as claimed in claim 20, characterized in that the tongues (10') are formed by parts bent out of the tubular or channel-shaped section (8, 17).

22. Insert member as claimed in claim 16, characterized in that the part forming the guiding channel (6) is designed in the manner of a straddling dowel (fig. 6).

23. Insert member as claimed in claim 17, characterized in that at least one opening (9) is provided within the flange (7) for passing therethrough a screw to be anchored within the bone (fig. 2, 3).

24. Insert member as claimed in claim 16, characterized in that its guiding channel (6) can be closed by a closure means.

25. Insert member as claimed in claim 24, characterized in that the closure means consists of a lid (18), preferably being provided with a collar (19) extending into the guiding channel (6).

26. Insert member as claimed in claim 25, characterized in that the lid (18) can be connected with the insert member (5) by means of a bayonet lock (22, 23).

27. Insert member as claimed in claim 25, characterized in that the lid (18) is provided with a thread (20) co-operating with a thread (21) provided in the insert member (5).

28. Insert member as claimed in claim 25, characterized in that the collar (19) of the lid (18) is conically shaped and is fixed within the guiding channel (6) by force-fitting.

29. Insert member as claimed in claim 24, characterized in that the guiding channel (6) can, after inserting the nails (4), at least partially be filled by casting with a hardening material, preferably a setting synthetic plastics material.

30. Insert member as claimed in claim 29, characterized in that the guiding channel (6) is provided with elevations, preferably grooves (16), within the portion to be filled by casting.

**Revendications**

1. Jeu d'instruments pour la réduction et l'immobilisation de fractures pertrochantériennes et subtrochantériennes, comportant au moins un clou (4) en matière élastique, courbé au moins en sa région terminale proximale, qui peut être introduit dans le canal médullaire (3) de l'os (1) en passant par un trou d'enfoncement disposé dans l'os (1), du côté proximal de l'articulation du genou et s'applique sous tension, en vertu de son élasticité, par le sommet de la courbure, contre la paroi du canal médullaire (3) qui est opposée au trou d'enfoncement et est muni à son extrémité distale d'une partie d'accouplement qui permet une liaison, solidaire en rotation de tous côtés, avec un instrument d'enfoncement, caractérisé en ce qu'il est prévu un élément d'insertion (5) pouvant s'insérer dans le trou d'enfoncement, qui présente un conduit de guidage (6) pour le clou (4) ou les clous (4) ou les clous, dont la paroi latérale subit la pression exercée sur l'os (1) par la partie d'accouplement du clou (4) placé sous tension dans le canal médullaire (3) de l'os (1) et la transmet à l'os avec répartition sur une plus grande surface, et qui est muni d'un dispositif de fixation (7, 9, 10, 14) empêchant son coulissement dans le trou d'enfoncement.

2. Jeu d'instruments selon la revendication 1, caractérisé en ce que l'élément d'insertion (5) est formé d'un tronçon tubulaire ou en forme de gouttière (8, 17) qui présente une bride s'écartant vers l'extérieur et s'appliquant contre le côté extérieur de l'os.

3. Jeu d'instruments selon les revendications 1 et 2, caractérisé en ce que l'axe (29) du tronçon tubulaire ou en forme de gouttière (8, 17) fait un angle aigu ($\alpha$) avec le plan de la bride.

4. Jeu d'instruments selon la revendication 1, caractérisé en ce que l'élément d'insertion (5) présente au moins un appendice (10, 10') s'appliquant contre la paroi du canal médullaire.

5. Jeu d'instruments selon la revendication 4, caractérisé en ce que l'appendice est formé par au moins une languette élastique (10, 10') (fig. 4, 5).

6. Jeu d'instruments selon la revendication 5, caractérisé en ce que les languettes (10') sont formées par des parties courbées hors du tronçon tubulaire ou en forme de gouttière (8, 17).

7. Jeu d'instruments selon la revendication 1, caractérisé en ce que la partie de l'élément d'insertion (5) qui forme le conduit de guidage (6) est constituée à la façon d'une cheville expansible (fig. 6).

8. Jeu d'instruments selon la revendication 2, caractérisé en ce que dans la bride (7) est prévue au moins une ouverture (9) pour le passage d'une vis pouvant s'ancrer dans l'os (fig. 2, 3).

9. Jeu d'instruments selon la revendication 1, caractérisé en ce que le conduit de guidage (6) de l'élément d'insertion (5) peut être fermé par un obturateur (18).

10. Jeu d'instruments selon la revendication 9, caractérisé en ce que l'obturateur est formé d'un couvercle (18) qui est, de préférence, muni d'un collet (19) pénétrant dans le conduit de guidage (6).

11. Jeu d'instruments selon la revendication 10, caractérisé en ce que le couvercle (18) peut être relié à l'élément d'insertion (5) par l'intermédiaire d'un verrouillage à baïonnette (22, 23).

12. Jeu d'instruments selon la revendication 10, caractérisé en ce que le couvercle (18) est muni d'un filetage (20) qui coopère avec un filetage (21) disposé dans l'élément d'insertion.

13. Jeu d'instruments selon la revendication 10, caractérisé en ce que le collet (19) du couvercle (18) est de forme conique et est fixé avec un ajustage serré dans le conduit de guidage (6).

14. Jeu d'instruments selon la revendication 9, caractérisé en ce que le conduit de guidage (6) peut, après l'insertion des clous (4), être comblé par coulée, au moins partiellement, par une matière durcissable, de préférence par une matière synthétique pouvant durcir complètement.

15. Jeu d'instruments selon la revendication 14, caractérisé en ce que le conduit de guidage (6) est muni, dans la partie à combler par coulée, de saillies, de préférence de cannelures (16).

16. Elément d'insertion pour l'utilisation dans un jeu d'instruments selon la revendication 1, caractérisé en ce qu'il présente un conduit de guidage (6) destiné au clou ou aux clous (4), dont la paroi latérale subit la pression exercée sur l'os par la partie d'accouplement du clou (4) placé sous tension dans le canal médullaire (3) de l'os (1) et la transmet à l'os (1) avec répartition sur une plus grande surface et qui est muni d'un dispositif de fixation (7, 9, 10, 14) empêchant son coulissement dans le trou d'enfoncement.

17. Elément d'insertion selon la revendication 16, caractérisé en ce qu'il présente un tronçon tubulaire ou en forme de gouttière (8, 17) et une bride (7) s'écartant de celui-ci vers l'extérieur, cette bride s'appliquant contre le côté extérieur de l'os.

18. Elément d'insertion selon la revendication 17, caractérisé en ce que l'axe (29) du tronçon tubulaire ou en forme de gouttière (8, 17) fait un angle aigu ($\alpha$) avec le plan de la bride.

19. Elément d'insertion selon la revendication 16, caractérisé en ce qu'il présente au moins un appendice (10, 10') s'appliquant contre la paroi du canal médullaire.

20. Elément d'insertion selon la revendication 19, caractérisé en ce que l'appendice est formé par au moins une languette élastique (10, 10') (fig. 4, 5).

21. Elément d'insertion selon la revendication 20, caractérisé en ce que les languettes (10') sont formées par des parties courbées hors du tronçon tubulaire ou en forme de gouttière (8, 17).

22. Elément d'insertion selon la revendication 16, caractérisé en ce que la partie, qui forme le conduit de guidage (6), est constituée à la façon d'une cheville expansible (fig. 6).

23. Elément d'insertion selon la revendication 17, caractérisé en ce que dans la bride (7) est prévue au moins une ouverture (9) pour le passage d'une vis pouvant s'ancrer dans l'os (fig. 2, 3).

24. Elément d'insertion selon la revendication 16, caractérisé en ce que son conduit de guidage (6) peut être fermé par un obturateur (18).

25. Elément d'insertion selon la revendication 24, caractérisé en ce que l'obturateur est formé d'un couvercle (18) qui est de préférence, muni d'un collet (19) pénétrant dans le conduit de guidage (6).

26. Elément d'insertion selon la revendication 15, caractérisé en ce que le couvercle (18) peut être relié à l'élément d'insertion (5) par l'intermédiaire d'un verrouillage à baïonnette (22, 23).

27. Elément d'insertion selon la revendication 25, caractérisé en ce que le couvercle (18) est muni d'un filetage (20) qui coopère avec un filetage (21) disposé dans l'élément d'insertion (5).

28. Elément d'insertion selon la revendication 25, caractérisé en ce que le collet (19) du couvercle (18) est de forme conique et est fixé avec un ajustage serré dans le conduit de guidage (6).

29. Elément d'insertion selon la revendication 24, caractérisé en ce que le conduit de guidage (6) peut, après l'insertion des clous (4), être comblé par coulée, au moins partiellement par une matière durcissable, de préférence par une matière synthétique pouvant durcir complètement.

30. Elément d'insertion selon la revendication 29, caractérisé en ce que le conduit de guidage (6) est muni, dans la partie à combler par coulée, de saillies, de préférence de cannelures (16).

# FIG.1

FIG.3

FIG.2

FIG.4

FIG.5

FIG.6

FIG.7